# EUROPEAN PATENT APPLICATION

(11) **EP 3 912 679 A1**
(43) Date of publication of application: **24.11.2021**
(21) Application number: 20175434.8
(22) Date of filing: 19.05.2020
(51) Int. Cl.: A61P 31/14, A61K 38/17

(54) **BBETA-15-42 FOR THE TREATMENT OF VIRAL ENDOTHELITIS**

(71) Applicant: Johann Wolfgang Goethe-Universität Frankfurt am Main, 60323 Frankfurt am Main (DE); Julius-Maximilians-Universität Würzburg, 97070 Würzburg (DE)
(72) Inventor: ZACHAROWSKI, Kai, 61350 Bad Homburg (DE); WUELFROTH, Petra, 8738 Uetliburg (CH); MEYBOHM, Patrick, 65779 Kelkheim (DE)
(74) Representative: Pföstl, Andreas

(57) **Abstract**

The invention is based on the fibrin-derived peptides as therapeutic compounds for the treatment of inflammatory complications of virally caused diseases, such as a diffuse inflammation of the endothelium, also known as systemic endotheliitis or vasculitis and related disorders. The use of certain fibrin derived peptides and analogues of these compounds resulted in a surprisingly effective patient recovery.

SEQUENCE LISTING

<110>  Johann Wolfgang Goethe-Universität Frankfurt am Main
       Julius-Maximilians-Universität Würzburg

<120>  COMPOUNDS FOR THE TREATMENT OF ENDOTHELIITIS IN CONTEXT OF VIRALLY
CAUSED DISEASES

<130>  U30999EP

<160>  28

<170>  PatentIn version 3.5

<210>  1
<211>  28
<212>  PRT
<213>  Artificial Sequence

<220>
<223>  fibrin-derived peptide B Beta 15-42

<400>  1

Gly His Arg Pro Leu Asp Lys Lys Arg Glu Glu Ala Pro Ser Leu Arg
1               5                   10                  15


Pro Ala Pro Pro Pro Ile Ser Gly Gly Gly Tyr Arg
            20                  25


<210>  2
<211>  29
<212>  PRT
<213>  Artificial Sequence

<220>
<223>  fibrin-derived peptide B Beta 15-42 derivative



<220>
<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (7)..(7)
<223>  any amino acid residue

<220>
<221>  misc_feature
<222>  (8)..(8)
<223>  any amino acid residue

<220>
<221>  misc_feature

<222>  (9)..(9)
<223>  any amino acid residue

<220>
<221>  misc_feature
<222>  (10)..(10)
<223>  any amino acid residue

<220>
<221>  misc_feature
<222>  (11)..(11)
<223>  any amino acid residue

<220>
<221>  misc_feature
<222>  (12)..(12)
<223>  any amino acid residue

<220>
<221>  misc_feature
<222>  (14)..(14)
<223>  any amino acid residue

<220>
<221>  misc_feature
<222>  (15)..(15)
<223>  any amino acid residue

<220>
<221>  misc_feature
<222>  (16)..(16)
<223>  any amino acid residue

<220>
<221>  misc_feature
<222>  (18)..(18)
<223>  any amino acid residue

<220>
<221>  misc_feature
<222>  (22)..(22)
<223>  any amino acid residue

<220>
<221>  misc_feature
<222>  (23)..(23)
<223>  any amino acid residue

<220>
<221>  misc_feature
<222>  (24)..(24)
<223>  any amino acid residue

<220>
<221>  misc_feature
<222>  (25)..(25)
<223>  any amino acid residue

<220>
<221>  misc_feature
<222>  (29)..(29)
<223>  OR1, wherein R1 is H or a C1-C10 alkyl group, or NR2R3, wherein
       R2 and R3 are hydrogen, a C1-C10 alkyl group, or -PEG5-60K, or a

residue NH-Y1-Z-PEG5-60K, wherein Y1 is a chemical bond or S, C,
K and R, and Z is a spacer

<400> 2

Gly His Arg Pro Xaa Xaa Xaa Xaa Xaa Xaa Xaa Xaa Pro Xaa Xaa Xaa
1               5                   10                  15

Pro Xaa Pro Pro Pro Xaa Xaa Xaa Xaa Gly Tyr Arg Xaa
            20                  25

<210> 3
<211> 29
<212> PRT
<213> Artificial Sequence

<220>
<223> fibrin-derived peptide B Beta 15-42 derivative

<220>
<221> misc_feature
<222> (5)..(5)
<223> any amino acid residue

<220>
<221> misc_feature
<222> (6)..(6)
<223> any amino acid residue

<220>
<221> misc_feature
<222> (7)..(7)
<223> any amino acid residue

<220>
<221> misc_feature
<222> (8)..(8)
<223> any amino acid residue

<220>
<221> misc_feature
<222> (9)..(9)
<223> any amino acid residue

<220>
<221> misc_feature
<222> (10)..(10)
<223> any amino acid residue

<220>
<221> misc_feature
<222> (11)..(11)
<223> any amino acid residue

<220>
<221> misc_feature
<222> (12)..(12)
<223> any amino acid residue

<220>

<221>  misc_feature
<222>  (14)..(14)
<223>  any amino acid residue

<220>
<221>  misc_feature
<222>  (15)..(15)
<223>  any amino acid residue

<220>
<221>  misc_feature
<222>  (16)..(16)
<223>  any amino acid residue

<220>
<221>  misc_feature
<222>  (18)..(18)
<223>  any amino acid residue

<220>
<221>  misc_feature
<222>  (22)..(22)
<223>  any amino acid residue

<220>
<221>  misc_feature
<222>  (23)..(23)
<223>  any amino acid residue

<220>
<221>  misc_feature
<222>  (24)..(24)
<223>  any amino acid residue

<220>
<221>  DISULFID
<222>  (25)..(25)
<223>  cysteine residue forms a disulfide bond with a second peptide
       having the same sequence

<220>
<221>  MISC_FEATURE
<222>  (29)..(29)
<223>  OR1, NR2R3, -PEG5-60K-CO-, NH-Y1-Z-PEG5-60K or
       NH-CH(CONH2)-(CH2)4-NH-CO-Y2-PEG5-60K, R1, R2, R3, R4 or R5 is H
       or a C1-C10 alkyl group, or R2 and R3 is -PEG5-60K, Y1 is a
       single bond or S, C, K and R, and Z is a spacer, Y2 is O or NH

<400>  3

Gly His Arg Pro Xaa Xaa Xaa Xaa Xaa Xaa Xaa Xaa Pro Xaa Xaa Xaa
1               5                   10                  15


Pro Xaa Pro Pro Pro Xaa Xaa Xaa Cys Gly Tyr Arg Xaa
            20                  25


<210>  4
<211>  29
<212>  PRT
<213>  Artificial Sequence

<220>
<223>  fibrin-derived peptide B Beta 15-42 derivative


<220>
<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (7)..(7)
<223>  any amino acid residue

<220>
<221>  misc_feature
<222>  (8)..(8)
<223>  any amino acid residue

<220>
<221>  misc_feature
<222>  (9)..(9)
<223>  any amino acid residue

<220>
<221>  misc_feature
<222>  (10)..(10)
<223>  any amino acid residue

<220>
<221>  misc_feature
<222>  (11)..(11)
<223>  any amino acid residue

<220>
<221>  misc_feature
<222>  (12)..(12)
<223>  any amino acid residue

<220>
<221>  misc_feature
<222>  (14)..(14)
<223>  any amino acid residue

<220>
<221>  misc_feature
<222>  (15)..(15)
<223>  any amino acid residue

<220>
<221>  misc_feature
<222>  (16)..(16)
<223>  any amino acid residue

<220>
<221>  misc_feature
<222>  (18)..(18)
<223>  Xaa can be any naturally occurring amino acid

<220>
<221>  misc_feature
<222>  (22)..(22)
<223>  any amino acid residue

<220>
<221>  misc_feature
<222>  (23)..(23)
<223>  any amino acid residue

<220>
<221>  DISULFID
<222>  (24)..(24)

<220>
<221>  misc_feature
<222>  (25)..(25)
<223>  any amino acid residue

<220>
<221>  misc_feature
<222>  (29)..(29)
<223>  OR1, NR2R3, -PEG5-60K-CO-, NH-Y1-Z-PEG5-60K or
      NH-CH(CONH2)-(CH2)4-NH-CO-Y2-PEG5-60K, R1, R2, R3, R4 or R5 is H
      or a C1-C10 alkyl group, or R2 and R3 is -PEG5-60K, Y1 is a
      single bond or S, C, K and R, and Z is a spacer, Y2 is O or NH

<400>  4

Gly His Arg Pro Xaa Xaa Xaa Xaa Xaa Xaa Xaa Xaa Pro Xaa Xaa Xaa
1               5                   10                  15


Pro Xaa Pro Pro Pro Xaa Xaa Cys Xaa Gly Tyr Arg Xaa
            20                  25


<210>  5
<211>  30
<212>  PRT
<213>  Artificial Sequence

<220>
<223>  fibrin-derived peptide B Beta 15-42 derivative


<220>
<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (7)..(7)
<223>  any amino acid residue

<220>

<221>  misc_feature
<222>  (8)..(8)
<223>  Xaa can be any naturally occurring amino acid

<220>
<221>  misc_feature
<222>  (9)..(9)
<223>  any amino acid residue

<220>
<221>  misc_feature
<222>  (10)..(10)
<223>  any amino acid residue

<220>
<221>  misc_feature
<222>  (11)..(11)
<223>  any amino acid residue

<220>
<221>  misc_feature
<222>  (12)..(12)
<223>  any amino acid residue

<220>
<221>  misc_feature
<222>  (14)..(14)
<223>  any amino acid residue

<220>
<221>  misc_feature
<222>  (15)..(15)
<223>  any amino acid residue

<220>
<221>  misc_feature
<222>  (16)..(16)
<223>  any amino acid residue

<220>
<221>  misc_feature
<222>  (18)..(18)
<223>  any amino acid residue

<220>
<221>  misc_feature
<222>  (22)..(22)
<223>  any amino acid residue

<220>
<221>  misc_feature
<222>  (23)..(23)
<223>  any amino acid residue

<220>
<221>  misc_feature
<222>  (24)..(24)
<223>  any amino acid residue

<220>
<221>  misc_feature
<222>  (25)..(25)
<223>  any amino acid residue

<220>
<221>  BINDING
<222>  (29)..(29)
<223>  dimer via lysine residue bound to -CO-(CH2)m-Y3-(CH2)m-CO-, Y3 is
      -N-CO-(CH2)n-NH-CO-Z-PEG5-60K or
      -N-CO-(CH2)n-NH-CO-CH(NH-CO-Z-PEG5-60K)-(CH2)4-NH-CO-Z-PEG5-60K,
      m is 1, 2, 3 or 4, Z is NH or O

<220>
<221>  misc_feature
<222>  (30)..(30)
<223>  is OH or NH2

<400>  5

Gly His Arg Pro Xaa Xaa Xaa Xaa Xaa Xaa Xaa Xaa Pro Xaa Xaa Xaa
1               5                   10                  15


Pro Xaa Pro Pro Pro Xaa Xaa Xaa Xaa Gly Tyr Arg Lys Xaa
            20                  25                  30


<210>  6
<211>  23
<212>  PRT
<213>  Artificial Sequence

<220>
<223>  fibrin-derived peptide B Beta 15-42 derivative


<220>
<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (8)..(8)
<223>  any amino acid residue

<220>
<221>  misc_feature
<222>  (9)..(9)
<223>  any amino acid residue

<220>
<221>  misc_feature
<222>  (10)..(10)
<223>  any amino acid residue

<220>
<221>  misc_feature
<222>  (12)..(12)
<223>  any amino acid residue

<220>
<221>  misc_feature
<222>  (16)..(16)
<223>  any amino acid residue

<220>
<221>  misc_feature
<222>  (17)..(17)
<223>  any amino acid residue

<220>
<221>  misc_feature
<222>  (18)..(18)
<223>  any amino acid residue

<220>
<221>  misc_feature
<222>  (19)..(19)
<223>  any amino acid residue

<220>
<221>  misc_feature
<222>  (20)..(20)
<223>  is a chemical bond or G

<220>
<221>  misc_feature
<222>  (21)..(21)
<223>  is a chemical bond or Y

<220>
<221>  misc_feature
<222>  (22)..(22)
<223>  is a chemical bond or R

<220>
<221>  misc_feature
<222>  (23)..(23)
<223>  OR1, wherein R1 is H or a C1-C10 alkyl group, or NR2R3, wherein
       R2 and R3 are hydrogen, a C1-C10 alkyl group, or -PEG5-60K, or a
       residue NH-Y1-Z-PEG5-60K, wherein Y1 is a chemical bond or S, C,
       K and R, and Z is a spacer

<400>  6

Gly His Arg Pro Xaa Xaa Pro Xaa Xaa Xaa Pro Xaa Pro Pro Pro Xaa
1               5                   10                  15

Xaa Xaa Xaa Xaa Xaa Xaa Xaa
            20

<210>  7
<211>  29
<212>  PRT
<213>  Artificial Sequence

<220>
<223>  fibrin-derived peptide B Beta 15-42 derivative

<220>

<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (7)..(7)
<223>  any amino acid residue

<220>
<221>  misc_feature
<222>  (8)..(8)
<223>  any amino acid residue

<220>
<221>  misc_feature
<222>  (9)..(9)
<223>  any amino acid residue

<220>
<221>  misc_feature
<222>  (10)..(10)
<223>  any amino acid residue

<220>
<221>  misc_feature
<222>  (11)..(11)
<223>  any amino acid residue

<220>
<221>  misc_feature
<222>  (12)..(12)
<223>  any amino acid residue

<220>
<221>  misc_feature
<222>  (14)..(14)
<223>  any amino acid residue

<220>
<221>  misc_feature
<222>  (15)..(15)
<223>  any amino acid residue

<220>
<221>  misc_feature
<222>  (16)..(16)
<223>  any amino acid residue

<220>
<221>  misc_feature
<222>  (18)..(18)
<223>  any amino acid residue

<220>
<221>  misc_feature
<222>  (22)..(22)
<223>  any amino acid residue

<220>
<221>  misc_feature
<222>  (23)..(23)
<223>  any amino acid residue

<220>
<221>  misc_feature
<222>  (24)..(24)
<223>  any amino acid residue

<220>
<221>  misc_feature
<222>  (25)..(25)
<223>  any amino acid residue

<220>
<221>  misc_feature
<222>  (26)..(26)
<223>  is a chemical bond or G

<220>
<221>  misc_feature
<222>  (27)..(27)
<223>  is a chemical bond or Y

<220>
<221>  misc_feature
<222>  (28)..(28)
<223>  is a chemical bond or R

<220>
<221>  misc_feature
<222>  (29)..(29)
<223>  OR1, wherein R1 is H or a C1-C10 alkyl group, or NR2R3, wherein
      R2 and R3 are hydrogen, a C1-C10 alkyl group, or -PEG5-60K, or a
      residue NH-Y1-Z-PEG5-60K, wherein Y1 is a chemical bond or S, C,
      K and R, and Z is a spacer

<400>  7

Gly His Arg Pro Xaa Xaa Xaa Xaa Xaa Xaa Xaa Xaa Pro Xaa Xaa Xaa
1               5                   10                  15

Pro Xaa Pro Pro Pro Xaa Xaa Xaa Xaa Xaa Xaa Xaa Xaa
            20                  25

<210>  8
<211>  16
<212>  PRT
<213>  Artificial Sequence

<220>
<223>  fibrin-derived peptide B Beta 15-42 derivative

<220>
<221>  misc_feature
<222>  (5)..(5)
<223>  any amino acid residue

<220>
<221>  misc_feature
<222>  (6)..(6)
<223>  any amino acid residue

<220>
<221>  misc_feature
<222>  (7)..(7)
<223>  any amino acid residue

<220>
<221>  misc_feature
<222>  (8)..(8)
<223>  an amino acid residue or a peptidomimetic element

<220>
<221>  misc_feature
<222>  (9)..(9)
<223>  any amino acid residue

<220>
<221>  misc_feature
<222>  (10)..(10)
<223>  any amino acid residue

<220>
<221>  misc_feature
<222>  (11)..(11)
<223>  any amino acid residue

<220>
<221>  misc_feature
<222>  (12)..(12)
<223>  any amino acid residue

<220>
<221>  misc_feature
<222>  (13)..(13)
<223>  any amino acid residue or a bond

<220>
<221>  misc_feature
<222>  (14)..(14)
<223>  any amino acid residue or a bond

<220>
<221>  misc_feature
<222>  (15)..(15)
<223>  any amino acid residue or a bond

<220>
<221>  misc_feature
<222>  (16)..(16)
<223>  OR1, wherein R1 is H or a C1-C10 alkyl group, or NR2R3, wherein
      R2 and R3 are hydrogen, a C1-C10 alkyl group, or -PEG5-60K, or a
      residue NH-Y1-Z-PEG5-60K, wherein Y1 is a chemical bond or S, C,
      K and R, and Z is a spacer

<400>  8

Gly His Arg Pro Xaa Xaa Xaa Xaa Xaa Xaa Xaa Xaa Xaa Xaa Xaa Xaa
1               5                   10                  15

<210>  9
<211>  28
<212>  PRT
<213>  Artificial Sequence

<220>
<223>  fibrin-derived peptide B Beta 15-42 derivative


<220>
<221>  misc_feature
<222>  (28)..(28)
<223>  modified to have a Cterminal amino group

<400>  9

Gly His Arg Pro Leu Asp Lys Lys Arg Glu Glu Ala Pro Ser Leu Arg
1               5                   10                  15


Pro Ala Pro Pro Pro Ile Ser Gly Gly Gly Tyr Arg
            20                  25


<210>  10
<211>  29
<212>  PRT
<213>  Artificial Sequence

<220>
<223>  fibrin-derived peptide B Beta 15-42 derivative


<220>
<221>  misc_feature
<222>  (29)..(29)
<223>  comprising a C-terminal -(S-CH2-CO-NH-PEG20K)-OH

<400>  10

Gly His Arg Pro Leu Asp Lys Lys Arg Glu Glu Ala Pro Ser Leu Arg
1               5                   10                  15


Pro Ala Pro Pro Pro Ile Ser Gly Gly Gly Tyr Arg Cys
            20                  25


<210>  11
<211>  29
<212>  PRT
<213>  Artificial Sequence

<220>
<223>  fibrin-derived peptide B Beta 15-42 derivative


<220>
<221>  misc_feature
<222>  (29)..(29)
<223>  comprising a C-terminal -(S-CH2-CO-NH-PEG20K)-amid

<400>  11

Gly His Arg Pro Leu Asp Lys Lys Arg Glu Glu Ala Pro Ser Leu Arg
1               5                   10                  15

Pro Ala Pro Pro Pro Ile Ser Gly Gly Gly Tyr Arg Cys
            20                  25

<210>  12
<211>  29
<212>  PRT
<213>  Artificial Sequence

<220>
<223>  fibrin-derived peptide B Beta 15-42 derivative

<220>
<221>  misc_feature
<222>  (26)..(26)
<223>  X is -(S-CH2-CO-NH-PEG20K)-

<220>
<221>  misc_feature
<222>  (29)..(29)
<223>  comprising a C-terminal amide group

<400>  12

Gly His Arg Pro Leu Asp Lys Lys Arg Glu Glu Ala Pro Ser Leu Arg
1               5                   10                  15

Pro Ala Pro Pro Pro Ile Ser Gly Cys Xaa Gly Tyr Arg
            20                  25

<210>  13
<211>  28
<212>  PRT
<213>  Artificial Sequence

<220>
<223>  fibrin-derived peptide B Beta 15-42 derivative

<220>
<221>  DISULFID
<222>  (25)..(25)
<223>  homodimeric peptide having a disulfide bond at position 25

<400>  13

Gly His Arg Pro Leu Asp Lys Lys Arg Glu Glu Ala Pro Ser Leu Arg
1               5                   10                  15

Pro Ala Pro Pro Pro Ile Ser Gly Cys Gly Tyr Arg
            20                  25

<210>  14
<211>  28
<212>  PRT
<213>  Artificial Sequence

<220>
<223>  fibrin-derived peptide B Beta 15-42 derivative

<220>
<221>  DISULFID
<222>  (24)..(24)
<223>  homodimeric peptide having a disulfide bond at position 24

<400>  14

Gly His Arg Pro Leu Asp Lys Lys Arg Glu Glu Ala Pro Ser Leu Arg
1               5                   10                  15

Pro Ala Pro Pro Pro Ile Ser Cys Gly Gly Tyr Arg
            20                  25

<210>  15
<211>  29
<212>  PRT
<213>  Artificial Sequence

<220>
<223>  fibrin-derived peptide B Beta 15-42 derivative

<220>
<221>  misc_feature
<222>  (25)..(25)
<223>  homodimer bound at position 25 via (S-CH2)2CH-CH2-CO-NH-PEG5-30K
       to each other

<220>
<221>  misc_feature
<222>  (29)..(29)
<223>  OR1, wherein R1 is H or a C1-C10 alkyl group, or NR2R3, wherein
       R2 and R3 are hydrogen, a C1-C10 alkyl group, or -PEG5-60K, or a
       residue NH-Y1-Z-PEG5-60K, wherein Y1 is a chemical bond or S, C,
       K and R, and Z is a spacer

<400>  15

Gly His Arg Pro Leu Asp Lys Lys Arg Glu Glu Ala Pro Ser Leu Arg
1               5                   10                  15

Pro Ala Pro Pro Pro Ile Ser Gly Cys Gly Tyr Arg Xaa
            20                  25

<210>  16
<211>  29
<212>  PRT
<213>  Artificial Sequence

<220>

<223>  fibrin-derived peptide B Beta 15-42 derivative

<220>
<221>  misc_feature
<222>  (24)..(24)
<223>  homodimer bound at position 25 via (S-CH2)2CH-CH2-CO-NH-PEG5-30K
      to each other

<220>
<221>  misc_feature
<222>  (29)..(29)
<223>  OR1, wherein R1 is H or a C1-C10 alkyl group, or NR2R3, wherein
      R2 and R3 are hydrogen, a C1-C10 alkyl group, or -PEG5-60K, or a
      residue NH-Y1-Z-PEG5-60K, wherein Y1 is a chemical bond or S, C,
      K and R, and Z is a spacer

<400>  16

Gly His Arg Pro Leu Asp Lys Lys Arg Glu Glu Ala Pro Ser Leu Arg
1               5                   10                  15

Pro Ala Pro Pro Pro Ile Ser Cys Gly Gly Tyr Arg Xaa
            20                  25

<210>  17
<211>  22
<212>  PRT
<213>  Artificial Sequence

<220>
<223>  fibrin-derived peptide B Beta 15-42 derivative

<220>
<221>  misc_feature
<222>  (22)..(22)
<223>  comprising a terminal OH group

<400>  17

Gly His Arg Pro Leu Ala Pro Ser Leu Arg Pro Ala Pro Pro Pro Ile
1               5                   10                  15

Ser Gly Gly Gly Tyr Arg
            20

<210>  18
<211>  22
<212>  PRT
<213>  Artificial Sequence

<220>
<223>  fibrin-derived peptide B Beta 15-42 derivative

<220>
<221>  misc_feature
<222>  (22)..(22)

<223>  comprising a C-terminal NH2 group

<400>  18

Gly His Arg Pro Leu Ala Pro Ser Leu Arg Pro Ala Pro Pro Pro Ile
1               5                   10                  15

Ser Gly Gly Gly Tyr Arg
            20

<210>  19
<211>  23
<212>  PRT
<213>  Artificial Sequence

<220>
<223>  fibrin-derived peptide B Beta 15-42 derivative

<220>
<221>  misc_feature
<222>  (23)..(23)
<223>  comprising a C-terminal -(S-succinimide-PEG20K)-OH

<400>  19

Gly His Arg Pro Leu Ala Pro Ser Leu Arg Pro Ala Pro Pro Pro Ile
1               5                   10                  15

Ser Gly Gly Gly Tyr Arg Cys
            20

<210>  20
<211>  23
<212>  PRT
<213>  Artificial Sequence

<220>
<223>  fibrin-derived peptide B Beta 15-42 derivative

<220>
<221>  misc_feature
<222>  (23)..(23)
<223>  comprising a C-terminal -(S-succinimido-PEG20K)-amide

<400>  20

Gly His Arg Pro Leu Ala Pro Ser Leu Arg Pro Ala Pro Pro Pro Ile
1               5                   10                  15

Ser Gly Gly Gly Tyr Arg Cys
            20

<210>  21
<211>  25
<212>  PRT

<213>  Artificial Sequence

<220>
<223>  fibrin-derived peptide B Beta 15-42 derivative


<220>
<221>  misc_feature
<222>  (25)..(25)
<223>  comprising a C-terminal -OH

<400>  21

Gly His Arg Pro Leu Asp Lys Lys Arg Glu Glu Ala Pro Ser Leu Arg
1               5                   10                  15


Pro Ala Pro Pro Pro Ile Ser Gly Gly
            20                  25


<210>  22
<211>  25
<212>  PRT
<213>  Artificial Sequence

<220>
<223>  fibrin-derived peptide B Beta 15-42 derivative


<220>
<221>  misc_feature
<222>  (25)..(25)
<223>  comprising a C-terminal -NH2

<400>  22

Gly His Arg Pro Leu Asp Lys Lys Arg Glu Glu Ala Pro Ser Leu Arg
1               5                   10                  15


Pro Ala Pro Pro Pro Ile Ser Gly Gly
            20                  25


<210>  23
<211>  26
<212>  PRT
<213>  Artificial Sequence

<220>
<223>  fibrin-derived peptide B Beta 15-42 derivative


<220>
<221>  misc_feature
<222>  (26)..(26)
<223>  comprising a C-terminal -OH

<400>  23

Gly His Arg Pro Leu Asp Lys Lys Arg Glu Glu Ala Pro Ser Leu Arg
1               5                   10                  15

Pro Ala Pro Pro Pro Ile Ser Gly Gly Gly
            20                  25

<210>  24
<211>  26
<212>  PRT
<213>  Artificial Sequence

<220>
<223>  fibrin-derived peptide B Beta 15-42 derivative

<220>
<221>  misc_feature
<222>  (26)..(26)
<223>  comprising a C-terminal -NH2

<400>  24

Gly His Arg Pro Leu Asp Lys Lys Arg Glu Glu Ala Pro Ser Leu Arg
1               5                   10                  15

Pro Ala Pro Pro Pro Ile Ser Gly Gly Gly
            20                  25

<210>  25
<211>  15
<212>  PRT
<213>  Artificial Sequence

<220>
<223>  fibrin-derived peptide B Beta 15-42 derivative

<220>
<221>  misc_feature
<222>  (8)..(8)
<223>  X is -(1S,2R)Achc-

<400>  25

Gly His Arg Pro Leu Asp Lys Xaa Ile Ser Gly Gly Gly Tyr Arg
1               5                   10                  15

<210>  26
<211>  15
<212>  PRT
<213>  Artificial Sequence

<220>
<223>  fibrin-derived peptide B Beta 15-42 derivative

<220>
<221>  misc_feature
<222>  (8)..(8)
<223>  X is Acdn

<400>  26

Gly His Arg Pro Leu Asp Lys Xaa Ile Ser Gly Gly Gly Tyr Arg
1               5                   10                  15


<210>  27
<211>  15
<212>  PRT
<213>  Artificial Sequence

<220>
<223>  fibrin-derived peptide B Beta 15-42 derivative


<220>
<221>  misc_feature
<222>  (8)..(8)
<223>  X is -(cis-4-Acha)-

<400>  27

Gly His Arg Pro Leu Asp Lys Xaa Ile Ser Gly Gly Gly Tyr Arg
1               5                   10                  15


<210>  28
<211>  15
<212>  PRT
<213>  Artificial Sequence

<220>
<223>  fibrin-derived peptide B Beta 15-42 derivative


<220>
<221>  misc_feature
<222>  (8)..(8)
<223>  X is -Haic-

<400>  28

Gly His Arg Pro Leu Asp Lys Xaa Ile Ser Gly Gly Gly Tyr Arg
1               5                   10                  15

## Description

### FIELD OF THE INVENTION

The invention is based on the fibrin-derived peptides as therapeutic compounds for the treatment of inflammatory complications of virally caused diseases, such as a diffuse inflammation of the endothelium, also known as systemic endotheliitis or vasculitis and related disorders. The use of certain fibrin derived peptides and analogues of these compounds resulted in a surprisingly effective patient recovery.

### DESCRIPTION

In December 2019, a cluster of viral pneumonia secondary to a novel coronavirus called Severe Acute Respiratory Syndrome Coronavirus 2 (SARS-CoV-2) occurred in China [1]. The clinical spectrum of this new disease varies widely and manifestations ranging from asymptomatic infections to severe progressive courses of the disease leading to respiratory failure or even death, have been observed [2]. Critically ill patients, which constitute about 2-9% of all infected patients, progress from pneumonia and hypoxemia to multi organ dysfunction, for which acute treatment options are scarce [3]. Gathering and disseminating information on potential experimental treatment options for this novel viral infection, for which no proven specific therapies are available, other than supportive care, is vital to enable prompt treatment. Currently, there is no clinical evidence supporting the efficacy and safety of a drug against any coronavirus in humans, including SARS-CoV-2, although many drugs show in vitro activity against different coronaviruses [4].

However, a number of off-label and compassionate use therapies have been tested worldwide, including chloroquine, hydroxychloroquine, azithromycin, lopinavir ritonavir, favipiravir, remedesivir, ribavirin, interferon, steroids and anti-IL-6 inhibitors, which demonstrate in vitro antiviral or anti-inflammatory properties. Yet, no proven effective therapy has been reported to date [5,6]. Thus, repurposing of already available, clinically safe and adverse reaction-tested and (ideally) approved drugs might be essential to rapidly treat COVID-19, and/or other viral disease, or their respectively caused inflammatory complications in infected patients.

FX-06 (or "FX06") is a peptide derived from the naturally occurring Bβ polypeptide chain (amino acids 15 to 42) of human fibrin, which binds the transmembrane adhesion receptor vascular endothelial (VE)-cadherin. Fibrin is an insoluble plasma protein generated by thrombin-mediated proteolytic cleavage from its soluble parent protein fibrinogen, a protein complex composed of three pairs of polypeptide chains, Aα, Bβ and γ chains. The proteolytic conversion of fibrinogen to fibrin releases two fibrinopeptides, A and B, derived from the Aα and Bβ polypeptide chains, respectively. The cleavage of fibrinopeptide B from the Bβ chain exposes amino acids 15 to 42 on the Bβ polypeptide chain of fibrinogen, thereby allowing the binding of fibrin and its degradation products to VE-cadherin.

Although the Bβ15-42 peptide occurs naturally in the blood, it has a lower affinity for the binding of VE-cadherin compared with the endogenous proinflammatory fibrin E1 fragment. However, the application of Bβ15-42 at supraphysiological doses was demonstrated to reduce leukocyte transmigration through the endothelial barrier, an effect that is associated with a reduction in infarct size in animal models of acute myocardial infarction. This finding led to the development of F X-06, which involves the administration of Bβ15-42 at supraphysiological doses. FX-06, in addition to binding to VE-cadherin, induced VE-cadherin-mediated intracellular signalling events in endothelial cells that stabilize the actin cytoskeleton and adherens-junctions. FX-06-induced VE-cadherin signalling could preserve the endothelial barrier function, thereby reducing capillary leakage; thus, FX-06 is also believed to be of potential therapeutic benefit in the treatment of diseases associated with a breakdown of the endothelial barrier. At the time of publication, FX-06 had completed a phase II clinical trial (ClinicalTrials.gov identifier: NCT00326976; FIRE) for the prevention of ischemia/reperfusion injury, and was undergoing preclinical assessment for the treatment of capillary leakage syndrome.

During outbreaks of Ebola virus in 2014, FX06 treatment was used as an empirical treatment in a patient with severe Ebola virus disease [7]. FX06 binds to the endothelial cells preventing leukocyte migration through the gap junctions of endothelial cells [8] preserving the integrity of the endothelium. FX06 is known for its anti-inflammatory properties [9,10] and was already investigated in clinical trials and has demonstrated convincing efficacy being well tolerated with a benign safety profile [11].

Thus, it is an objection of the invention to repurpose a drug under clinical investigation for other related diseases for the treatment of virally caused inflammatory complications, in particular in the context of the present COVID-19 pandemic.

### BRIEF DESCRIPTION OF THE INVENTION

Generally, and by way of brief description, the main aspects of the present invention can be described as follows:
In **a first aspect**, the invention pertains to a compound for use in the treatment of an inflammatory disorder of the endothelium in a subject, wherein the compound is a fibrin-derived peptide B Beta 15-42 and/or at least one functional derivative thereof or a physiologically acceptable salt thereof, and wherein the treatment comprises administering the compound in a therapeutically effective amount to the subject.
In **a second aspect**, the invention pertains to a compound for use in the treatment of an inflammatory complication induced or caused by a viral infection and/or viral disease, in a subject, wherein the compound is a fibrin-derived peptide B Beta 15-42 and/or at least one functional derivative thereof or a physiologically acceptable salt thereof, and wherein the treatment comprises administering the compound in a therapeutically effective amount to the subject.
In **a third aspect**, the invention pertains method of treatment of an inflammatory disorder of the endothelium in a subject, wherein the treatment comprises a step of administering a therapeutically effective amount of a fibrin-derived peptide B Beta 15-42 and/or at least one functional derivative thereof or a physiologically acceptable salt thereof, to the subject.
In **a fourth aspect**, the invention pertains the invention pertains method of treatment of an inflammatory complication induced or caused by a viral infection and/or viral disease in a subject, wherein the treatment comprises a step of administering a therapeutically effective amount of a fibrin-derived peptide B Beta 15-42 and/or at least one functional derivative thereof or a physiologically acceptable salt thereof, to the subject.
In **a fifth aspect**, the present invention further pertains to the use of a fibrin-derived peptide B Beta 15-42 and/or at least one functional derivative thereof or a physiologically acceptable salt thereof, in the manufacture of a medicament for the treatment of one of the disorders and/or complications of the first to the fourth aspect of the invention.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect**, the invention pertains to a compound for use in the treatment of an inflammatory disorder of the endothelium in a subject, wherein the compound is a fibrin-derived peptide B Beta 15-42 and/or at least one functional derivative thereof or a physiologically acceptable salt thereof, and wherein the treatment comprises administering the compound in a therapeutically effective amount to the subject.

In one alternative first aspect, and as may be further described, defined, claimed or otherwise disclosed herein, the invention relates to a method for the treatment of an inflammatory disorder of the endothelium in a subject, the method comprising administering a a fibrin-derived peptide B Beta 15-42 and/or at least one functional derivative thereof or a physiologically acceptable salt thereof, to the subject.

In another related first aspect, and as may be further described, defined, claimed or otherwise disclosed herein, the invention pertains to a use of a fibrin-derived peptide B Beta 15-42 and/or at least one functional derivative thereof or a physiologically acceptable salt thereof for the manufacture of a medicament for the treatment of a inflammatory disorder of the endothelium in a subject.

The terms "subject," "individual," "host," and "patient" are used interchangeably herein to refer to a mammal, including, but not limited to, murines (rats, mice), felines, non-human primates (e.g., simians), humans, canines, ungulates, etc. In some embodiments, a "subject" is a human, and can also be referred to as a "patient."

As used herein, the term "endothelium" refers to a layer of cells that line the inside surfaces of blood vessels and form capillaries. The term "endothelial cell" refers to the specialized cells that form the epithelium endothelium and line the inner walls of blood vessels. As such an endothelium in some embodiments refers to one or more endothelial cells of the vascular system. An inflammation of the endothelium which is systemic or systemic endotheliitis.

In some preferred embodiments of the invention the inflammatory disorder of the endothelium is a diffuse inflammation of the endothelium, and preferably is endotheliitis (or vasculitis).

In a further embodiment the inflammatory disorder of the endothelium, and/or inflammatory complication associated with a viral disease - such as COVID 19 - may be vasculitis, e.g. systemic vasculitis. The term "vasculitis" indicates that blood vessels are inflamed. The consequences of vasculitis depend on the size, site and number of blood vessels involved. Infarction of the tissue that the blood vessel supplies may occur when small or medium-sized arteries are involved (e.g. coronary artery vasculitis can result in a heart attack) but the effects are less serious when very small blood vessels such as capillaries are involved. An exception is when there is extensive local vasculitis, such as can occur in the kidney, resulting in glomerulonephritis. A vasculitis of the invention may be a small vessel vasculitis (Wegener's granulomatosis, Churg-Strauss syndrome, microscopic poly-angiitis, Henoch-Schoenlein purpura, essential cryoglobulin-aemic angiitis), medium-sized vessel vasculitis (cutaneous leucocytoclastic angiitis) and large vessel vasculitis (Poly-arteritis nodosa, Kawasaki's disease, Giant cell (temporal) arteritis, Takayasu's arteritis).

A "therapeutically effective amount" or "efficacious amount" means the amount of a compound (such as a fibrin-derived peptide B Beta 15-42 and/or at least one functional derivative thereof or a physiologically acceptable salt thereof) that, when administered to a mammal or other subject for treating a disease, is sufficient to effect such treatment for the disease. The "therapeutically effective amount" will vary depending on the compound, the disease and its severity and the age, weight, etc., of the subject to be treated.

In some embodiments, an "effective amount" of a fibrin-derived peptide B Beta 15-42 and/or at least one functional derivative thereof or a physiologically acceptable salt thereof is an amount that, when administered in one or more doses to an individual having an inflammatory disorder of the endothelium, e.g., endotheliitis. For example, in some embodiments, an "effective amount" of a fibrin-derived peptide B Beta 15-42 and/or at least one functional derivative thereof or a physiologically acceptable salt thereof is an amount that, when administered in one or more doses to an individual having an inflammatory disorder of the endothelium, e.g., endotheliitis, is effective to reduce such inflammation in one or more cells of the endothelium of the subject treated.

The present disclosure provides compounds for use in, and methods for, treating an inflammatory complication of the endothelium that is associated with a virus infection, where the virus is a member of the *Filoviridae,* or *Coronaviridae* family, the method involving administering an effective amount of the fibrin-derived peptide B Beta 15-42 peptide, or a derivative or salt thereof, to an individual (subject) infected with a member of the *Coronaviridae* family. *Coronaviridae* includes, e.g., coronaviruses, e.g., human coronavirus 229E (HCoV- 229E), human coronavirus OC43 (HCoV-OC43), Middle Eastern Respiratory (MERS) Virus and SARS-CoV (the causative agent of severe acute respiratory syndrome (SARS)), which cause upper respiratory tract infection, lower respiratory tract infections, and gastroenteritis.

According to a preferred embodiment of the present invention it is possible to administer fibrin-derived peptide B Beta 15-42 as the sole active ingredient or in combination with at least one derivative thereof. Of course, it is also possible to administer at least one derivative of fibrin-derived peptide B Beta 15-42 as the sole active ingredient or in combination with fibrin-derived peptide B Beta 15-42.

Fibrin-derived peptide B Beta 15-42 and/or at least one derivative thereof may be administered to a subject in need thereof by any means known to be suitable to those skilled in the art, including intravenous, subcutaneous, intramuscular and mucosal administration. A formulation may thus be formulated for administration via the intravenous, subcutaneous, intramuscular and mucosal route. The mucosal route may be exemplified by, but is not limited to, the pulmonary, nasal, sublingual or buccal route.

For intravenous, subcutaneous or intramuscular administration, the formulation may be provided as a sterile solution, suspension or an emulsion. The formulation may be applied by means of an injection or an infusion. For mucosal administration, the formulation may be provided as an aqueous spray and may be applied directly by means of a spray container or an inhalator. Alternatively, the formulation may be administered to the mucosa as an aqueous gel.

Fibrin-derived peptide B Beta 15-42 and/or at least one derivative thereof may be provided in a liquid or solid form. Both components may be part of a lyophilisate (solid form) which can be combined with a buffer or a physiological salt solution prior administration to a subject. Alternatively, the components may be part of a liquid formulation which may include oils, polymers, vitamins, carbohydrates, amino acids, salts, buffers, albumin, surfactants, or bulking agents. Exemplary carbohydrates include sugar or sugar alcohols such as mono-, di-or polysaccharides, or water-soluble glucans. The saccharides or glucans can include fructose, dextrose, lactose, glucose, mannose, sorbose, xylose, maltose, sucrose, dextran, pullulan, dextrin, alpha- and beta-cyclodextrin, soluble starch, hydroxethyl starch and carboxymethylcellulose, or mixtures thereof. "Sugar alcohol" is defined as a C4 to C8 hydrocarbon having an -OH group and includes galactitol, inositol, mannitol, xylitol, sorbitol, glycerol, and arabitol. These sugars or sugar alcohols mentioned above may be used individually or in combination. In some embodiments, one can use a buffer in the composition to minimize pH changes in the solution before lyophilization or after reconstitution. Any physiological buffer may be used, but in some cases can be selected form citrate, phosphate, succinate, and glutamate buffers or mixtures thereof.

The inventive compounds show an effect at a dose ranging from 0.001 mg/kg body weight to 500 mg/kg body weight, preferably at a dose ranging from 0.1 mg/kg to 50 mg/kg.

In other embodiments of the fibrin-derived peptide B Beta 15-42 and/or at least one derivative thereof is administered to the subject in a daily dose of 100mg to 1000mg, preferably 150mg to 800mg, more preferably 200mg to 600mg, and most preferably between 300mg and 500mg, and preferably is administered to the subject in a daily dose of about 300mg, about 400mg, about 500mg or about 600mg. Furthermore, in additional or alternative embodiments, the fibrin-derived peptide B Beta 15-42 and/or at least one derivative thereof is administered in single daily doses or, preferably, in two separate (preferably equivalent) daily doses within an interval of one hour, preferably 30 min, most preferably 10 minutes. The administration in some embodiment to the subject may proceed via intravenous (bolus) injection or via intravenous infusion, for example infusion of the therapeutically effective amount over a time of 1 to 6 hours. In another alternative embodiment, the administration of the therapeutically effective amount of the compound of the invention proceeds via continuous infusion over a time period of 1 to 10, preferably about 3 to 6 hours.

The herein indicated treatment of a subject with a fibrin-derived peptide according to the invention, or any derivative or salt thereof, proceeds for a time sufficient to reduce the inflammation of the endothelium, or as least for a time inflammatory signs start to normalize , but in preferred embodiments the treatment continues for 1 to 10 days, and preferably for 3 to 7 days.

In any one of the herein disclosed embodiments, the individual is a human of from about one month to about 6 months, from about 6 months to about 1 year, from about 1 year to about 5 years, from about 5 years to about 12 years, from about 13 years to about 18 years, from about 18 years to about 25 years, from about 25 years to about 50 years, from about 50 years to about 75 years of age, or older than 75 years of age. In some embodiments, the individual has a chronic lung disease (e.g., emphysema, chronic bronchitis, asthma, cystic fibrosis, bronchiectasis, COPD, or interstitial lung disease) or other risk for poor prognosis such as a cardio-vascular disorder, diabetes or obesity. In some embodiments, the individual has, in addition to a coronavirus infection, pneumonia, where the pneumonia is caused by the coronavirus (preferably SARS-CoV-2) or by a bacterial infection. In some embodiments the human subject is immunocompromised.

A subject to be treated with a fibrin-derived peptide according to the invention, or any derivative or salt thereof, is in some preferred embodiments distinguished by any one or any combination of the following clinical parameters:
- A corona virus infection, preferably a SARS-CoV2, infection;
- A PaO₂/FᵢO₂ < 300; and/or
- Having received an endotracheal intubation and mechanical ventilation within 72 h before starting the treatment;
- A (serum) value of LDH >365 U/L
- A (serum) value of high sensitivity CRP > 40 mg/ml, and/or
- A lymphocyte count < 15%.

In context of the invention the fibrin-derived peptide B Beta 15-42 is a peptide that preferably consists of amino acid sequence GHRPLDKKREEAPSLRPAPPPISGGGYR (SEQ ID No. 1). Such peptide will be referred to in the present disclosure as "FX06" or "FX06-peptide".

In context of the herein disclosed invention a "derivative" of FX06 is preferably a peptide having not more than 3, preferably 2, more preferably not more than one amino acid substitution, deletion, and/or addition compared to the amino acid sequence shown in SEQ ID NO: 1, and preferably wherein such substitution, deletion, and/or addition does not affect the first 4 amino acid positions of SEQ ID NO: 1.

Other derivatives of FX06 considered to be comprised in the present disclosure are derivatives and analogues of FX06 specifically disclosed in WO 2019/011879. IN the following there is provided a similar description of such derivatives:

A "derivative" of fibrin-derived peptide B Beta 15-42 may comprise one or more amino acid changes compared to the wild-type peptide. Preferably the first four amino acid residues of the derivative are identical to the wild-type peptide. The N-terminal end of the derivative is preferably a free amino group whereas the C-terminal end may be modified by the addition of organic groups like polyethylene glycol (PEG). The derivative of the present invention may comprise 1, 2, 3, 4 5, 6, 7, 8, 9, or 10 amino acid exchanges compared to the wild-type peptide. The derivative may be a monomer, dimer, trimer or tetramer of a modified or wild-type fibrin-derived peptide B Beta 15-42 whereby the monomers of the aforementioned multimers are linked to each other by disulphide bonds of cysteine residues, for instance, or other chemical moieties forming a bridge between the monomers. The derivatives of the present invention exhibit properties for inhibiting, treating and/or preventing an inflammatory disorder of the endothelium, and/or an inflammatory complication associated with a viral disease, such as COVID-19.

Further, and in some embodiments preferred, derivatives of fibrin-derived peptide B Beta 15-42 are known in the art. For instance, WO 2019/011879, WO 2007/095659, WO 2007/095660, WO 2007/095661, WO 2009/137850, WO 2009/137851 and WO 2009/137852 disclose derivatives of fibrin-derived peptide B Beta 15-42, which can be used in the medical applications subject of the present invention.

According to a further preferred embodiment of the present invention the fibrin-derived peptide B Beta 15-42 derivative has a general formula selected from the group consisting of:
H₂N-GHRP X₁X₂X₃X₄X₅X₆X₇X₈PX₉ X₁₀ X₁₁PX₁₂PPPX₁₃X₁₄X₁₅X₁₆GYR-X₁₇ (I), (SEQ ID No. 2), 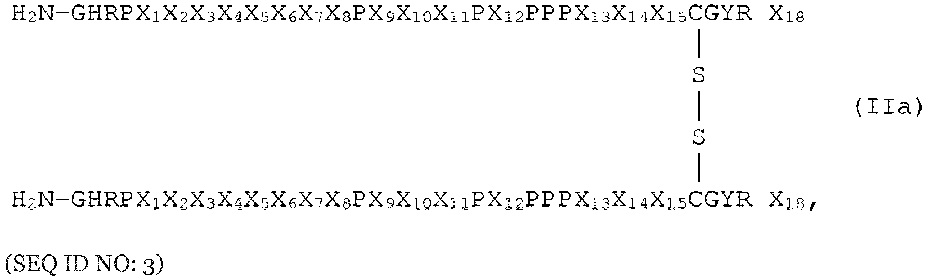 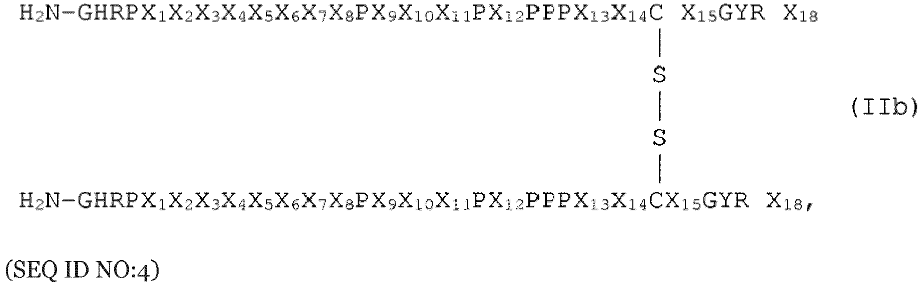 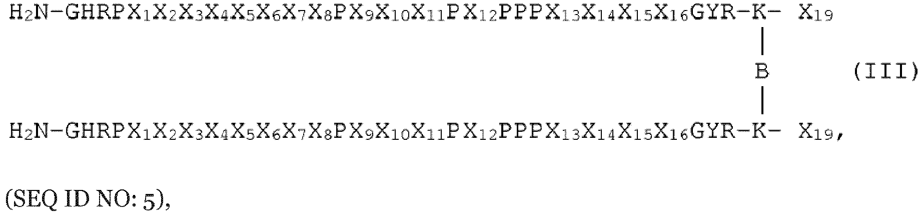
H₂N-GHRPX₁X₈PX₉X₁₀X₁₁PX₁₂PPPX₁₃X₁₄X₁₅X₁₆B (1)B (2)B (3)-X₁₇ (IV), (SEQ ID No. 6)
H₂N-GHRPX₁X₂X₃X₄X₅X₆X₇X₈PX₉X₁₀X₁₁PX₁₂PPPX₁₃X₁₄X₁₅X₁₆B (1)B (2)B (3)-X₁₇ (V) (SEQ ID No. 7)
   and
H₂N-GHRPX₂₀X₂₁X₂₂-p-X₂₃X₂₄X₂₅X₂₆X₂₇X₂₈X₂₉-X₁₇ (VI), (SEQ ID No. 8)
   wherein
   X₁, X₂, X₃, X₄, X₅, X₆, X₇, X₈, X₉, X₁₀, X₁₁, X₁₂, X₁₃, X₁₄, X₁₅ and X₁₆ are independently selected from the group of amino acid residues,
   X₁₇ is
      a) OR₁, wherein R₁ is hydrogen or a C₁-C₁₀ alkyl group, or
      b) NR₂R₃, wherein R₂ and R₃ are independently hydrogen, a C₁-C₁₀ alkyl group, or
      c) a residue -PEG_{5-60K}, wherein the PEG-residue is linked to the N atom via a spacer, or
      d) a residue NH-Y₁-Z-PEG_{5-60K}, wherein Y₁ is a chemical bond or an amino acid residue selected from the group consisting of S, C, K and R, and Z is a spacer by way of which a polyethylene glycol (PEG)-residue is linked,
   or X₁₈ is
      a) OR₁, wherein R₁ is hydrogen or a C₁-C₁₀ alkyl group, or
      b) NR₂R₃, wherein R₂ and R₃ are independently hydrogen, a C₁-C₁₀ alkyl group, or
      c) a residue -PEG_{5-60K}, wherein the PEG-residue is linked to the N atom via a spacer, or
      d) a residue NH-Y₁-Z-PEG_{5-60K}, wherein Y₁ is a chemical bond or an amino acid residue selected from the group consisting of S, C, K and R, and Z is a spacer by way of which a polyethylene glycol (PEG)-residue is linked, or
      e) a residue -PEG_{5-60K}-CO-NR₄R₅, wherein R₄ and R₅ are independently hydrogen or a C₁-C₁₀ alkyl group, or
      f) a residue NH-CH(CONH₂)-(CH₂)₄-NH-CO-Y₂-PEG_{5-60K}, wherein Y₂ is an oxygen atom or an NH group,
   X₁₉ is OH or NH₂,
   X₂₀, X₂₁, X₂₂, X₂₃, X₂₄, X₂₅ and X₂₆ are independently selected from the group consisting amino acid residues,
   X₂₇, X₂₈ and X₂₉ are independently selected from the group consisting amino acid residues or are independently a single bond
   B is -CO-(CH₂)ₘ-Y₃-(CH₂)ₘ-CO-bound via the CO group to the ε amino group of amino acid residue K, wherein m is an integer from 1 to 4 and Y₃ is -N-CO-(CH₂)ₙ-NH-CO-Z-PEG_{5-60K} or
      wherein n is an integer from 1 to 4 and Z is NH or O,
      B(1) is a chemical bond or G,
      B(2) is a chemical bond or Y,
      B(3) is a chemical bond or R,
   β is an amino acid residue or a peptidomimetic element, wherein said amino acid residue is selected from the group consisting of L-proline, D-proline, L-hydroxyproline, D-hydroxyproline, L-(O-benzyl)-hydroxyproline, D-(O-benzyl)-hydroxyproline, L-(O-tert. butyl)-hydroxyproline, 4-(O-2-naphtyl) -hydroxyproline, 4-(O-2-naphtyl-methyl)-hydroxyproline, 4-(O-phenyl)-hydroxyproline, 4-(4-phenyl-benzyl)-proline, cis-3-phenyl-proline, cis-4-phenyl-proline, trans-4-phenyl-proline, cis-5-phenyl-proline, trans-5-phenyl-proline, 4-benzyl-proline, 4-bromobenzyl-proline, 4-cyclohexylproline, 4-fluor-proline, L-tetrahydroisoquinoline-2-carboxylic acid (L-Tic), all diastereomers of octahydro- indole-2-carboxylic acid (Oic), and all diastereomers of 1-aza-bicyclo[3,3,0]octane-2-carboxylic acid, and wherein said peptidomimetic element is selected from the group consisting of cis-2-amino-cyclopentane carboxylic acid (cis-Acpc), (1R, 2R)-(2-aminocyclopentane carboxylic acid ((1R, 2R)-Acpc), (1S, 2S)-(2-aminocyclopentane carboxylic acid ((1S, 2S)-Acpc), 1-aminomethyl-cyclohexane acetic acid (1-Achc), 3-amino-1-carboxymethyl-pyridin-2-one (Acpo), 1-amino-cyclobutanecarboxylic acid (1-Acbc), 1-amino-cyclohexane-carboxylic acid (1-Achc), cis-4-amino-cyclohexane-acetic acid (4-Acha), (1R, 2R)-2-aminocyclohexane carboxylic acid ((1R, 2R)-Ache), (1R, 2S)-2-aminocyclohexane carboxylic acid ((1R, 2S)-Ache), (1S,2R)-2-aminocyclohexane carboxylic acid ((1S, 2R)-Achc), (1S, 2S)-2-aminocyclohexane carboxylic acid ((1S, 2S)-Ache), 1-amino-cyclopentane carboxylic acid (1-Acpec), 1-amino-cyclopropane carboxylic acid (1-Acprc), 4-(2-aminoethyl)-6-dibenzof uranpropionic acid (Aedfp), (R, S)-i-aminoindane-i-carboxylic acid (1-Aic), 2-aminoindane-2-carboxylic acid (2-Aic), 2'-(aminomethyl)-biphenyl-2-carboxylic acid (Ambc), 2-aminomethyl-phenylacetic acid (Ampa), 3-amino-2-naphthoic acid (Anc), 4-amino-tetrahydropyran-4-carboxylic acid (Atpc), (R, S)-2-aminotetraline-2-carboxylic acid (2-Atc), (2S,6S,9S)-6-amino-2-carboxymethyl-3,8-diazabicyclo-[4,3,0] -nonane-1,4-dione (Acdn), (R)-3-amino-5-carboxymethyl-2, 3-dihydro-1,5-benzo-thiazepin-4 (5H)-one (Acbt), (S)-3-amino-5-carboxymethyl-2,3-dihydro-1, 5-benzoxazepin-4 (5H)-one (Acbo), (R, S)-3-amino-1-carboxymethyl-,23,4,5-tetrahydro-1H-[1]-benzazepin-2-one (1-Acmb), (S)-4-amino-2-carboxymethyl-1, 3, 4, 5-tetrahydro-2H-[2]-benzazepin-3-one (2-Acmb), (R, S)-3-amino-1-carboxymethyl-valerolactame (Acmv), 3-(2-aminoethyl)-1-carboxymethyl-quinazoline-2, 4-dione (Acq), (2S,5S)-5-amino-i, 2, 4, 5, 6, 7-hexahydro-azepino [3,2,1-hi] -indole-4-one-2-carboxylic acid (Haic), (R, S)-3-amino-N-1-carboxymethyl-2-oxo-5-cyclohexyl-1,4-benzodiazepine (Accb), (R, S)-3-amino-N-1-carboxymethyl-2-oxo-5-phenyl-1, 4-benzodiazepine (Acpb), (2S,11aS)-2-amino-10-carboxymethyl-1, 2,3, 11a-tetrahydro-10H-pyrrolo [2, 1-c] [1, 4] -benzodiazepine-5, 11-dione (PBD), (2S,3'S)-2-(4'-(3'-benzyl-2'-oxo-piperazin-1-yl))-3-phenylpropionic acid (Bppp), 3-carboxymethyl-1-phenyl-1,3,8-tria-zaspiro [4.5]decan-4-one (Cptd), (R, S)-3 -amino-9-Boc-1,2,3, 4-tetrahydro-carbazole-3-carboxylic acid (The), 3-exo-amino-bicyclo [2.2.1]heptane-2-exo-carboxylic acid (Abhc), (3S)-3-Amino-1-carboxymethylcaprolactam (Accl), (S,S)-(ProLeu) spirolactamePhe (PLSP) and 2-Oxo-3-amino-7-thia-1-azabicyclo [4. 3. o]nonane-9-carboxylic acid (BTD).

According to a preferred embodiment of the present invention
X₁₅ or X₁₆ of formula (I) is an amino acid selected from the group consisting of C and K, which is linked to residue Z-PEG_{5-60K} via the heteroatom in the side chain, and wherein
X₁₇ of formula (I) is
a) OR₁, wherein R₁ is hydrogen or a C₁-C₁₀-alkyl group, or
b) NR₂R₃, wherein R₂ and R₃ are independently hydrogen or a C₁-C₁₀ alkyl group.

According to another preferred embodiment of the present invention
X₁, X₉ , X₁₀, X₁₄, X₂₀ and X₂₃ are independently L, I, S, M or A,
X₂, X₆, X₇ and X₂₁ are independently E or D,
X₃, X₄, X₅ X₁₁ and X₂₂ are independently R or K
X₈ X₁₂, X₂₄, X₂₅ and X₂₆ are independently A, G, S, or
L,
X₁₃ is I, L or V,
X₁₅ and X₁₆ of formula (IIa), (IIb) are independently G, A, S, or C,
X₂₇ is G, A or L,
X₂₈ is Y, F, H or a single chemical bond and X₂₉ is R, K or a single chemical bond.

According to a preferred embodiment of the present invention a compound of formula I is preferably selected from the group consisting of GHRPLDKKREEAPSLRPAPPPISGGGYR-N H₂ (SEQ ID No. 9), GHRPLDKKREEAPSLRPAPPPISGGGYRC-(SC H₂-CO-NH-PEG_{20K}) -OH (SEQ ID No. 10), GHRPLDKKREEAPSLRPAPPPISGGGYRC(S-C H₂-CO-NH-PEG_{20K}) -amid (SEQ ID No. 11) and GHRPLDKKREEAPSLRPAPPPISGC(S-CH₂-CO-NH-PEG_{20K})-GYR-amid (SEQ ID No. 12).

According to another preferred embodiment of the present invention a compound of formula Ila is preferably (GHRPLDKKREEAPSLRPAPPPISGCGYR) 2 a Cys25-Cys25 homodimeric cysteine peptide (SEQ ID No. 13).

According to another preferred embodiment of the present invention a compound of formula lib is (GHRPLDKKREEAPSLRPAPPPISCGGYR) 2 a Cys24-Cys24 homodimeric cysteine peptide (SEQ ID No. 14).

According to a further preferred embodiment of the present invention a compound of the formula III is: 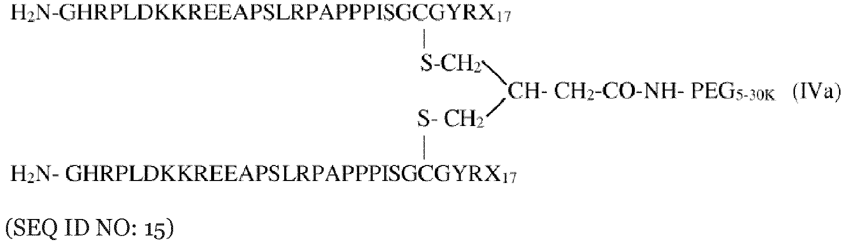 or

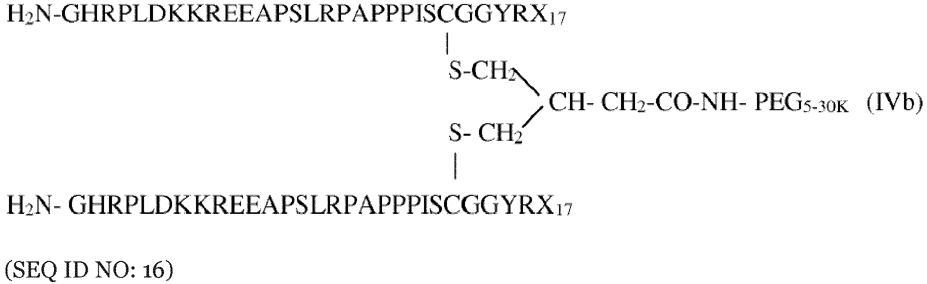

In another preferred embodiment of the present invention a compound of formula IV is selected from the group consisting of GHRPLAPSLRPAPPPISGGGYR -OH (SEQ ID No. 17), GHRPLAPSLRPAPPPISGGGYR -NH2 (SEQ ID No. 18), GHRPLAPSLRPAPPPISGGGYRC(S-succinimide-PEG_{20K}) -OH (SEQ ID No. 19) and GHRPLAPSLRPAPPPISGGGYRC-(S-succinimidoPEG_{20K}) -amide (SEQ ID No. 20).

In another preferred embodiment of the present invention a compound of formula V is selected from the group consisting of GHRPLDKKREEAPSLRPAPPPISGG-OH (SEQ ID No.21), GHRPLDKKREEAPSLRPAPPPISGG-N H₂ (SEQ ID No. 22), GHRPLDKKREEAPSLRPAPPPISGGG-OH (SEQ ID No. 23) and GHRPLDKKREEAPSLRPAPPPISGGG-N H₂ (SEQ ID No. 24).

In a further preferred embodiment of the present invention a compound of formula VI is selected from the group consisting of GHRPLDK-(1S,2R)Achc-ISGGGYR (SEQ ID No. 25), GHRPLDK-Acdn-ISGGGYR (SEQ ID No. 26), GHRPLDK(cis-4-Acha)-ISGGGYR (SEQ ID No. 27) and GHRPLDK-Haic-ISGGGYR (SEQ ID No. 28).

In **a second aspect**, the invention pertains to a compound for use in the treatment of an inflammatory complication induced or caused by a viral infection and/or viral disease, in a subject, wherein the compound is a fibrin-derived peptide B Beta 15-42 and/or at least one functional derivative thereof or a physiologically acceptable salt thereof, and wherein the treatment comprises administering the compound in a therapeutically effective amount to the subject.

The above disclosure, explanations, definitions and embodiments equally apply for the second aspect where appropriate.

The term "inflammatory complication induced or caused by a viral infection and/or viral disease" as used in context of the herein disclosed invention pertains to a secondary disorder, complication or side effect that is caused by a viral infection. Such complication treatable in accordance with the herein disclosed invention is of an inflammatory nature, and preferably involves or is associated with a diffuse inflammation of the endothelium (see above). Preferably, the complication treatable by the invention is an endotheliitis or vasculitis according to the descriptions elsewhere herein.

In **a third aspect**, the invention pertains method of treatment of an inflammatory disorder of the endothelium in a subject, wherein the treatment comprises a step of administering a therapeutically effective amount of a fibrin-derived peptide B Beta 15-42 and/or at least one functional derivative thereof or a physiologically acceptable salt thereof, to the subject.

The above disclosure, explanations, definitions and embodiments equally apply for the third aspect where appropriate.

In **a fourth aspect**, the invention pertains the invention pertains method of treatment of an inflammatory complication induced or caused by a viral infection and/or viral disease in a subject, wherein the treatment comprises a step of administering a therapeutically effective amount of a fibrin-derived peptide B Beta 15-42 and/or at least one functional derivative thereof or a physiologically acceptable salt thereof, to the subject.

The above disclosure, explanations, definitions and embodiments equally apply for the fourth aspect where appropriate.

In **a fifth aspect**, the present invention further pertains to the use of a fibrin-derived peptide B Beta 15-42 and/or at least one functional derivative thereof or a physiologically acceptable salt thereof, in the manufacture of a medicament for the treatment of one of the disorders and/or complications of the first to the fourth aspect of the invention.

The above disclosure, explanations, definitions and embodiments equally apply for the fifth aspect where appropriate.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1****:** shows norepinephrine dosages in all patients of example 1 over 14 days since admission
**Figure 2****:** shows oxygenation index in all patients of example 1 over 14 days since admission
**Figure 3****:** shows chest X-ray at day 3 (left), 7 (middle) and 14 (right) of patient 1 of example 1
**Figure 4****:** shows chest X-ray at day 1 (left), 6 (middle) and 14 (right) of patient 2 of example 1
**Figure 5****:** shows chest X-ray at day 1 (left), 6 (middle) and 10 (right) of patient 3 of example 1
**Figure 6****:** shows inflammatory markers in all patients of example 1 over 14 days since admission; A: c-reactive protein; B: Interleukin 6 (IL-6); C: pro-calcitonin .
**Figure 7****:** shows a chest X-rays at day 0 (left), 4 (centre), and 7 (right) after first FX06 administration (patient 1) of example 2.
**Figure 8****:** shows the ventilation parameters of patient 1 of example 2
**Figure 9****:** shows chest X-rays on day 1 (left) and 4 (right) after start of FX06 treatment (patient 2 of example 2).
**Figure 10****:** shows ventilation parameters of patient 2 of example 2
**Figure 11****:** shows serum inflammation markers of patient 2 of example 2
**Figure 12****:** shows ventilation and oxygenation parameters of patient 3 of example 2
**Figure 13****:** shows serum inflammation markers of patient 3 of example 2
**Figure 14****:** shows chest X-rays on day 2 (left) and 8 (right) after first FX06 application (patient 3 of example 2)

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

Therefore, in the herein disclosed examples, FX06 as a therapeutic agent in a case series involving critically ill patients is discussed, presenting with associated hypoxemia and multi organ dysfunction in two university tertiary centres in Germany.

The examples show:

### Example 1: Critically ill patients suffering from a infection in a first university care centre.

### Cases

All patients were diagnosed positive for SARS-CoV-2 using naso- and oropharyngeal swab samples, amplifying the beta coronavirus E gene and the specific SARS-CoV-2 RdRp gene by PCR.

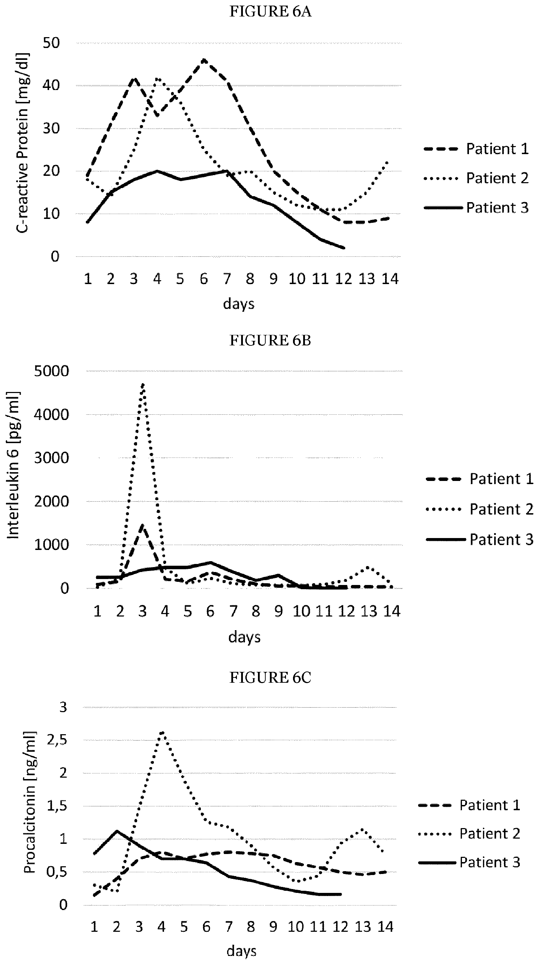

The demographic, clinical characteristics at admission and treatment of the patients are shown in Table 1. The course of inflammatory markers is presented in figure 6. Upon admission to the intensive care unit, all patients received mechanical ventilation and critical care therapy as put forth by Poston et al. [12].

**Table 1: Demographics and clinical characteristics at admission and treatment**

| | Patient 1 | Patient 2 | Patient 3 | Reference range |
|---|---|---|---|---|
| Age (y) | 52 | 78 | 63 | |
| Gender | male | male | male | |
| BMI | 31 | 35 | 26 | |
| Comorbidities | no | coronary artery disease, arterial hypertension | bronchial asthma | |
| Invasive ventilation | yes | yes | yes | |
| Severity of the infection at admission | moderate | moderate | moderate | |
| Antibiotic therapy | Imipeneme | Imipeneme | Imipeneme, Voriconazol | |
| Days on ICU prior to FX06 treatment | 0 | 3 | 4 | |
| Daily dose of FX06 | 500mg | 6oomg | 400mg | |
| Duration of FX06 treatment (days) | 7 | 7 | 4 | |
| vv- ECMO therapy | yes | no | yes | |

| Outcome | still at hospital | death | still at hospital | |
|---|---|---|---|---|
| *Laboratory results at admission* | | | | |
| White Blood cell count (cells per 10⁶/L) | 14.02 | 15.56 | 6.26 | 3.92-9.81 |
| Lymphocyte (cells per 10⁶/L) | 1.12 | 1.24 | 0.71 | 1.05 - 3.24 |
| Platelets | 320 | 147 | 171 | 146 - 328 |
| LDH U/L | 378 | 1277 | 417 | < 248 |
| Kreatinin mg/dL | 0.72 | 2.34 | 0.43 | 0.7 - 1.2 |
| C-reactive Protein (mg/dL) | 20.13 | 18.08 | 8.00 | < 0.5 |
| Ferritin ng/mL | 883 | 5505 | 3708 | 18 - 360 |
| Procalcitonin ng/mL | 0.15 | 0.30 | 0.78 | < 0.5 |
| Lactate mg/dL | 9.0 | 14 | 9.0 | 4.5-14.5 |
| IL-6 pg/mL | 92.3 | 25.4 | 250 | < 7 |
| D-Dimer ng/mL | 629 | 130136 | 1056 | < 500 |
| aPTT (sec) | 28 | 30 | 29 | 25-37 |
| SAPS II Score | 57 | 75 | 43 | |
| PₐO_{2/}FᵢO₂ ratio | 186 | 141 | 131 | |

| | | | | |
|---|---|---|---|---|
| Y = years, BMI = Body mass index, LDH = lactate dehydrogenase, U = Units, aPTT = activated partial Thromboplastin time, SAPS II = Simplified Acute Physiology Score; PaO₂ = partial pressure arterial oxygen, FiO₂ = fraction of inspired oxygen, vv = venovenous, ECMO = extracorporeal membrane oxygenation. | | | | |

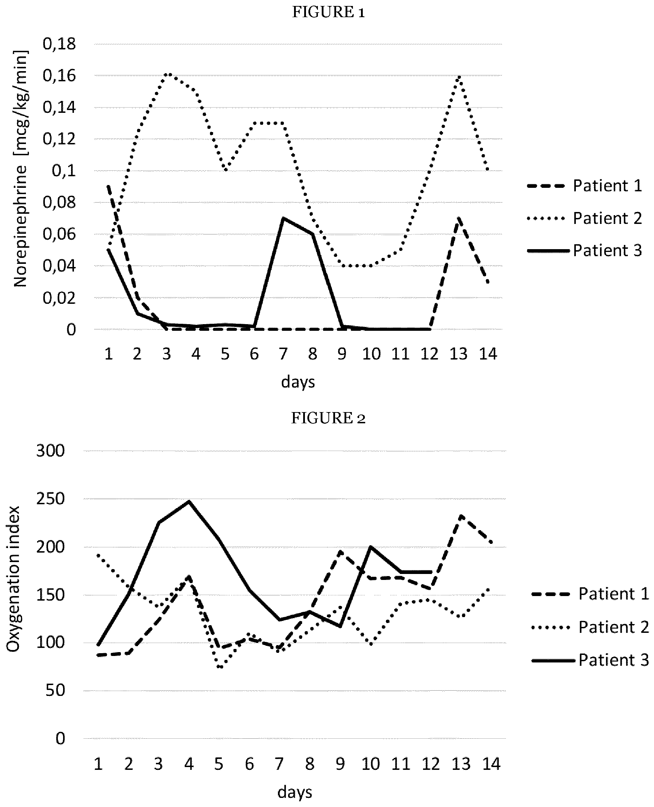

Patient 1 presented to the emergency department with symptoms typical for infection (fever, cough and dyspnea). Chest CT scan showed bilateral, multiple ground-glass opacities with peripheral lung and subpleural distribution in the upper and lower lobes. Due to severe respiratory failure and hypoxemia, mechanical ventilation was initiated immediately after hospital admission. Furthermore, venovenous ECMO therapy was started on hospital day 2 due to refractory hypoxemia. The patient was treated with FX06 for 7 days from the day of admission. Radiographic findings showed regressive lung consolidations over the following 7 days and the patient's clinical condition improved significantly (figure 1-3). On hospital day 14, the patient was decannulated from ECMO. The patient is still being treated in our intensive care unit.

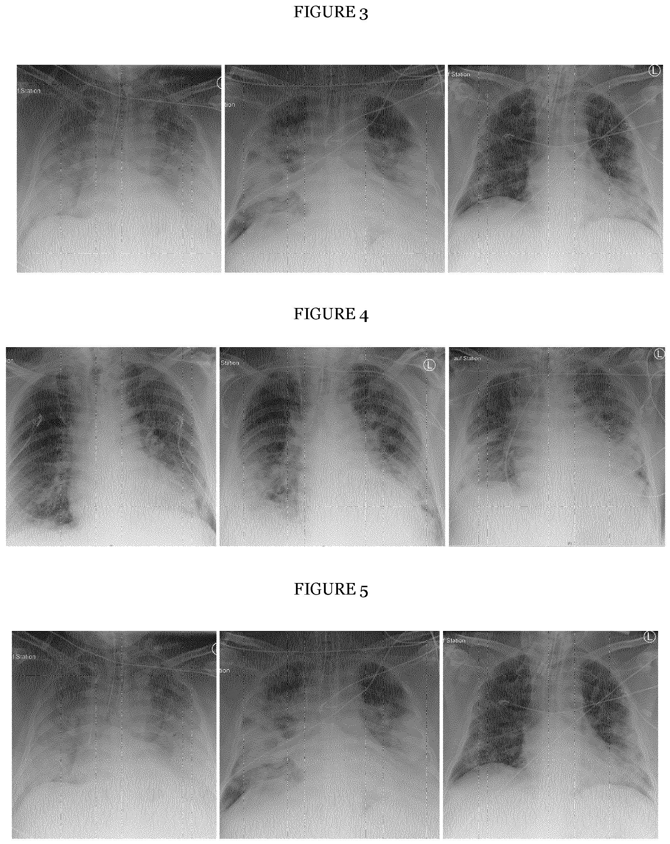

Similar to patient 1, patient 2 presented with cough, fever and dyspnea to the emergency department and a rapid respiratory deterioration occurred. Mechanical ventilation was initiated 2 hours after hospital admission. Radiographic findings revealed typical features for infection. The patient received FX06 treatment for 7 days from day of admission. Initially the clinical condition improved in terms of increased oxygenation and reduction of vasopressor therapy (figure 1-2) but radiographic findings revealed a deterioration of pulmonary infiltrates (figure 4). Microbiological analyses identified bacterial co-infections of Aspergillus fumigatus and Serratia marcescens, which were treated with corresponding antibacterial and antifungal drugs. The patient presented a continuous improvement of his condition and received a percutaneous tracheotomy on hospital day 18. On hospital day 23 he developed a fulminant septic shock leading to severe multi organ failure and death within 24h.

Patient 3 was transferred from a secondary care hospital to our university tertiary hospital on illness day 8 because of a deterioration of his clinical condition. Due to severe hypoxemia mechanical ventilation was necessary and ECMO treatment as well as FX06 therapy was initiated on hospital day 2. Radiographic diagnostic demonstrated acute lung injury and nearly "white lungs" (figure 5). Within one week after FX06 treatment a significant improvement of pulmonary infiltrates was observed. Inflammatory markers decreased continuously and oxygenation improved over time, but weaning from ECMO was not successful until now (hospital day 12). The patient is still being treated in our intensive care unit.

### Conclusion

The results of example 1 report a successful administration of FX06 in three critically ill patients suffering from COVID-19-associated severe ARDS. In all patients, a significant improvement following the FX06 administration was observed. Given that fulminant septic adverse events, as observed in patient 3, represent typical complications in critically ill patients, this is not associated with the therapy of FX06. Hence, the example demonstrates the use of FX06 in severe associated ARDS as an effective therapy to improve the course of the disease and in particular in ameliorating the inflammatory complications associated with COVID-19.

### Example 2: Critically ill patients suffering from a infection in a second university care centre.

To this date, we treated three patients in individualized therapeutic attempts with FX06 in the second university care center. Patient 1 was a 51 years-old female with a medical history of obesity, arterial hypertension, and rheumatoid arthritis with immunosuppressive therapy. Patient 2 was a 71 years-old male with a medical history of type 2 diabetes. Patient 3 was a 55 years-old male with a history of arterial hypertension and obesity.

All three patients were diagnosed with SARS-CoV-2 pneumonia resulting in severe acute respiratory distress syndrome (ARDS) and required extracorporeal membrane oxygenation (ECMO) therapy upon or early during their treatment in our facility. All patients were referred to our hospital from basic care providers due to the severity of their conditions. All patients required ECMO therapy at the time of FX06 treatment.

FX06 400 mg was administered once per day after informed consent by the close relative.

**Table 2: FX06 Dosing of Patients:**

| **Patients** | **Days on ICU** | **Doses** |
|---|---|---|
| | **before first FX06** | |
| 1 | 10 | 3 x 400mg (=3 days) |
| 2 | 22 | 4 x 400mg (=4 days) |
| 3 | 2 | 4 x 400mg (=4 days) |

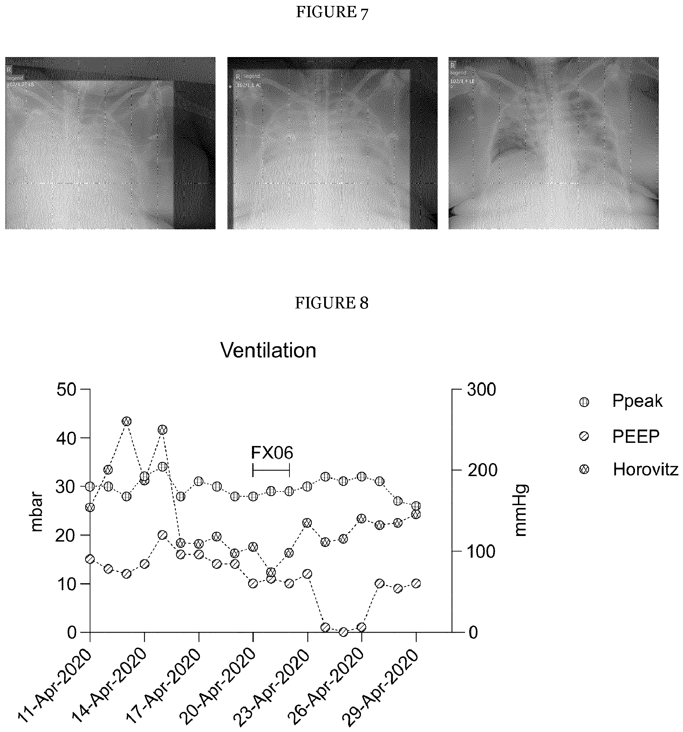

Patient 1 presented with near-complete lung failure four days after admission, with only 40 ml of tidal volume left. Six days after first dose of FX06, pulmonary infiltrations started to regress significantly (fig. 7). Accordingly, ventilation parameters improved, too, with increased oxygenation indices (Horovitz, s. Fig.8). Although the COVID-19-induced ARDS was significantly improving after the FX06 treatment period, the patient unfortunately went into multi-organ failure and died of acute liver failure several days later.

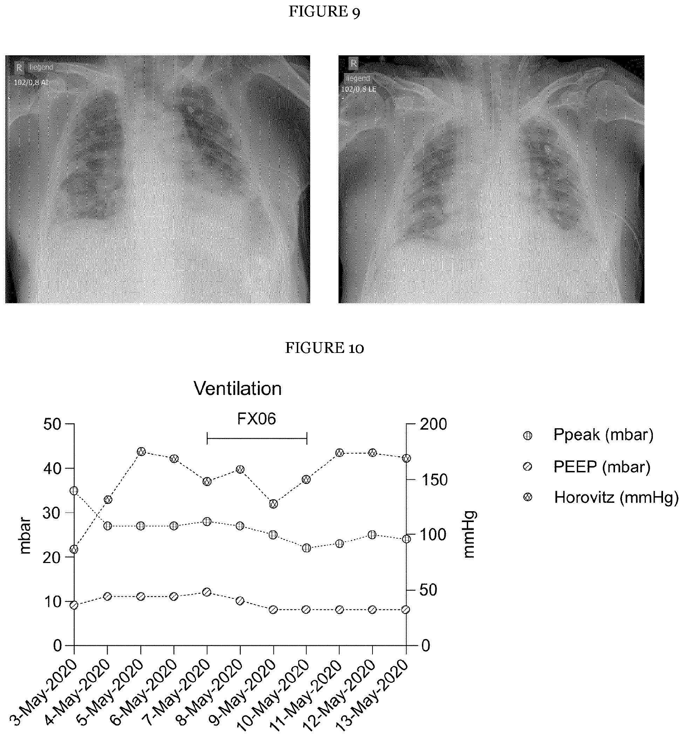

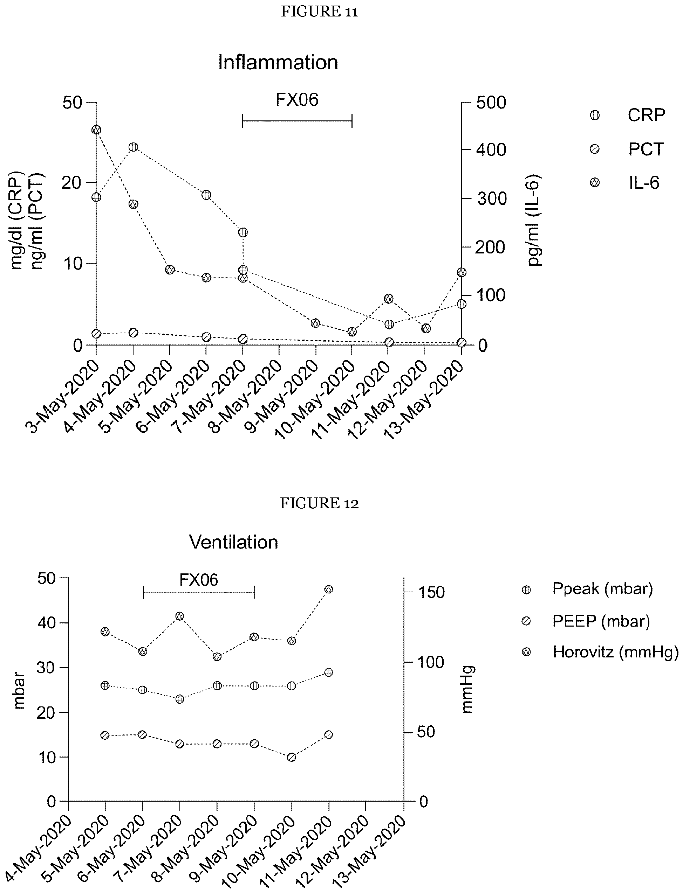

Patient 2 showed a less rapid onset of respiratory failure, with the need for mechanical ventilation no sooner than 8 days after initial hospitalization compared to only one day in patients 1 and 3. After the beginning of FX06 treatment, pulmonary infiltrations also started to ease as seen in Fig. 9. Additionally, ventilation was improved (Fig. 10) and serum inflammation markers fell (Fig. 11) in light of the FX06 treatment. Patient 2 currently is still being treated in our intensive care unit.

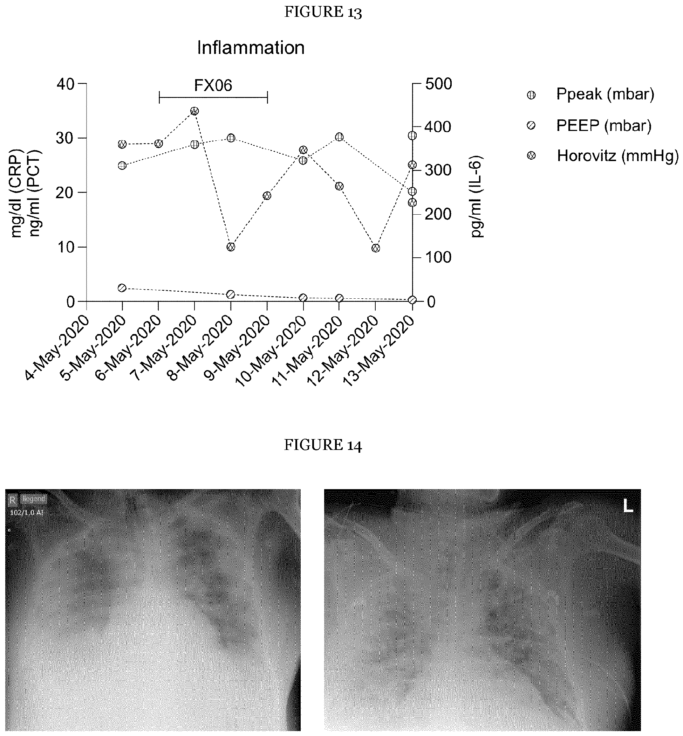

Patient 3 presented with a similar response as patient 2 to the treatment with FX06. Ventilation and oxygenation improved after the intervention (Fig. 12) and serum inflammation markers were slowly regressive (Fig. 13). Also like with both other patients, chest X-rays showed easing of pulmonary infiltrations (Fig. 14). Patient 3 is also still in intensive care treatment.

Example 2 therefore demonstrates a successful administration of FX06 in three critically ill patients suffering from COVID-19-associated severe ARDS. In all three patients, substantial features of the complex syndrome improved over the course and in the days following the treatment. There is probably no link between the administration of FX06 and patient 1's death, but rather attribute it to the severity of the underlying condition in conjunction with the patient's immunosuppressive therapy due to a pre-existing condition. Based on the data of the 3 patients FX06 is shown to be safe to use in severe ARDS and is capable of improving distinct features of the disease and in particular its inflammatory complications such as endotheliitis.

### REFERENCES

The references are:
1. Huang C, Wang Y, Li X, Ren L, Zhao J, Hu Y, Zhang L, Fan G, Xu J, Gu X: Clinical features of patients infected with 2019 novel coronavirus in Wuhan, China. The lancet 2020; 395: 497-506
2. Zhou F, Yu T, Du R, Fan G, Liu Y, Liu Z, Xiang J, Wang Y, Song B, Gu X: Clinical course and risk factors for mortality of adult inpatients with in Wuhan, China: a retrospective cohort study. The lancet 2020
3. Xu Z, Shi L, Wang Y, Zhang J, Huang L, Zhang C, Liu S, Zhao P, Liu H, Zhu L: Pathological findings of associated with acute respiratory distress syndrome. The Lancet respiratory medicine 2020; 8: 420-422
4. Kalil AC: Treating COVID-19-Off-Label Drug Use, Compassionate Use, and Randomized Clinical Trials During Pandemics. JAMA 2020
5. Magagnoli J, Narendran S, Pereira F, Cummings T, Hardin JW, Sutton SS, Ambati J: Outcomes of hydroxychloroquine usage in United States veterans hospitalized with Covid-19. medRxiv 2020: 2020.04.16.20065920
6. Horby P, Cao B, Wang Y, Wang C: Evaluation of the Efficacy and Safety of Intravenous Remdesivir in Adult Patients with Severe Pneumonia caused by virus Infection: study protocol for a Phase 3 Randomized, Double-blind, Placebo-controlled, Multicentre trial. 2020
7. Wolf T, Kann G, Becker S, Stephan C, Brodt H-R, de Leuw P, Grünewald T, Vogl T, Kempf VA, Keppler OT: Severe Ebola virus disease with vascular leakage and multiorgan failure: treatment of a patient in intensive care. The Lancet 2015; 385: 1428-1435
8. Bergt S, Gruenewald M, Beltschany C, Grub A, Neumann T, Albrecht M, Vollmar B, Zacharowski K, Roesner JP, Meybohm P: The fibrin-derived peptide Bβ15-42 (FX06) ameliorates vascular leakage and improves survival and neurocognitive recovery: implications from two animal models of cardiopulmonary resuscitation. Critical care medicine 2016; 44: e988-e995
9. Ahrens I, Peter K: FX-06, a fibrin-derived Bβ. Current Opinion in Investigational Drugs 2009; 10
10. Henning R, Zacharowski K, Petzelbauer P: FX06 (fibrin-derived peptide Bbeta15-42)-A potential candidate for myocardial reperfusion therapy. Drugs of the Future 2006; 31: 811-818
11. Atar D, Petzelbauer P, Schwitter J, Huber K, Rensing B, Kasprzak JD, Butter C, Grip L, Hansen PR, Süselbeck T: Effect of intravenous FX06 as an adjunct to primary percutaneous coronary intervention for acute ST-segment elevation myocardial infarction: results of the FIRE (Efficacy of FX06 in the Prevention of Myocardial Reperfusion Injury) trial. Journal of the American College of Cardiology 2009; 53: 720-729
12. Poston JT, Patel BK, Davis AM: Management of critically ill adults with COVID-19. Jama 2020

## Claims

1. **A compound for use in the treatment of an inflammatory disorder and/or complication of the endothelium caused by a viral infection** in a subject, wherein the compound is a fibrin-derived peptide B Beta 15-42 and/or at least one functional derivative thereof or a physiologically acceptable salt thereof, and wherein the treatment comprises administering the compound in a therapeutically effective amount to the subject.

2. The compound for use of to claim 1, wherein the fibrin- derived peptide B Beta 15-42 consists of amino acid sequence GHRPLDKKREEAPSLRPAPPPISGGGYR (SEQ ID No. 1).

3. The compound for use of any one of claims 1 or 2, wherein the viral infection is caused by a virus that infects endothelial cells.

4. The compound for use of any one of claims 1 to 3, wherein the inflammatory disorder of the endothelium involves an accumulation of inflammatory cells associated with the endothelium in the subject.

5. The compound for use of any one of claims 1 to 4, wherein the viral infection in the subject is caused by a virus of the family of *Coronaviridae.*

6. The compound for use of any one of claims 1 to 5, wherein the subject suffers from one or more disorders increasing the risk for a poor prognosis, preferably selected from the group of an immunocompromising disorder, a chronic lung disease, hypertension, obesity, and diabetes

7. The compound for use of claim 6, wherein the chronic lung disease is asthma, chronic obstructive pulmonary disease, cystic fibrosis, interstitial lung disease, bronchitis, sarcoidosis, idiopathic pulmonary fibrosis, bronchiectasis, acute respiratory distress syndrome, or acute lung injury.

8. The compound for use of any one of claims 1 to 7, wherein the viral infection is caused by HCoV-229E, HcoV-OC43 (HCoV-OC43), Middle East respiratory syndrome-related coronavirus (MERS)-CoV or severe acute respiratory syndrome coronavirus (SARS-CoV), and preferably is caused by SARS Cov-2.

9. The compound for use of any one of claims 1 to 8, wherein the compound is administered to the subject in a daily dose of 100mg to 1000mg, preferably 150mg to 800mg, more preferably 200mg to 600mg, and most preferably between 300mg and 500mg, and preferably is administered to the subject in a daily dose of about 300mg, about 400mg, about 500mg or about 600mg.

10. The compound for use of any one of the preceding claims, wherein the compound is administered to the subject via intravenous (bolus) injection or via intravenous infusion.

11. The compound for use of any one of the preceding claims, wherein the treatment involves a therapy for 1 to 10 day, preferably of 3 to 7 days.

12. The compound for use of any one of the preceding claims, wherein the compound is administered in a dose between 0.1 mg/kg body weight to 50 mg/kg body weight, and preferably 0.5 to 20 mg/kg body weight, and most preferably 0.7 to 17.5 mg/kg body weight.

13. The compound for use according to any one of the preceding claims, wherein the compound is formulated as a pharmaceutical composition comprising the compound together with a pharmaceutically acceptable carrier and/or excipient.
